(19)

**Europäisches Patentamt**
**European Patent Office**
**Office européen des brevets**

(11) **EP 4 763 088 A1**

(12) **EUROPEAN PATENT APPLICATION**

(43) Date of publication:
**24.06.2026 Bulletin 2026/26**

(21) Application number: **24383410.8**

(22) Date of filing: **19.12.2024**

(51) International Patent Classification (IPC):
**A61B 5/361** (2021.01)

(52) Cooperative Patent Classification (CPC):
**A61B 5/361**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Fundación para la Investigación Sanitaria del Hospital Gregorio Marañón 28007 Madrid (ES)**

(72) Inventors:
• **Ríos Muñoz, Gonzalo  Ricardo**
  **28007 Madrid (ES)**
• **Arenal Maíz, Ángel**
  **28007 Madrid (ES)**

(74) Representative: **Clarke, Modet y Cía., S.L. C/ Suero de Quiñones 34-36 28002 Madrid (ES)**

(54) **SYSTEM AND COMPUTER-IMPLEMENTED METHOD FOR DETECTING ATRIAL FIBRILLATION DRIVERS**

(57)     A system and computer-implemented method for detecting atrial fibrillation drivers in signals that exhibit automatic auto-accelerated activity. The system (1) comprises a detection unit (2) configured to obtain sets (16) of bipolar EGMs corresponding to a multielectrode catheter acquisition; calculate cycle lengths (105) and detect activity intervals (107) in each bipolar EGM (20); classify the activity intervals (107) into discrete intervals (109) when their duration is lower than an activity threshold, or otherwise into highfrequency complex activity, HFCA, intervals (111); calculate an entanglement index (113) for each HFCA interval (111) that depends on the detection, within an entanglement time window (31) that at least partially includes the HFCA interval (111), of one or more discrete intervals (109) in other bipolar EGMs (20) of the set (16) of bipolar EGMs (20) with a reduction in the cycle length (105) during the HFCA interval (111); and detect atrial fibrillation drivers (7) in each acquisition based on the entanglement indexes (113). The invention improves the precision of AF driver identification.

EP 4 763 088 A1

100

RECEIVE SETS OF UNIPOLAR ATRIAL EGMs STORED IN A DATA STORAGE UNIT AND CORRESPONDING TO MULTI-ELECTRODE CATHETER ACQUISITIONS — 101

OBTAIN A SET OF BIPOLAR EGMs — 102 ⇨ SET OF BIPOLAR EGMs — 16

CALCULATE CYCLE LENGTHS IN EACH BIPOLAR EGM — 104 ⇨ CYCLE LENGTHS — 105

DETECT ACTIVITY INTERVALS IN EACH BIPOLAR EGM — 106 ⇨ ACTIVITY INTERVALS — 107

CLASSIFY ACTIVITY INTERVALS INTO DISCRETE INTERVALS AND HFCA INTERVALS BASED ON THE DURATION OF THE ACTIVITY INTERVAL — 108 ⇨ DISCRETE INTERVALS — 109 / HFCA INTERVALS — 111

CALCULATE ENTANGLEMENT INDEX FOR EACH HFCA INTERVAL BASED ON THE DETECTION OF DISCRETE INTERVALS IN OTHER BIPOLAR EGMs WITH A REDUCED CYCLE LENGTH — 110 ⇨ ENTANGLEMENT INDEXES — 113

DETECT AF DRIVERS IN THE HFCA INTERVALS BASED ON THEIR ENTANGLEMENT INDEXES — 112

AF DRIVERS — 7

FIG. 3

## Description

## FIELD

The present invention is comprised within the field of systems and methods used to detect atrial fibrillation drivers.

## BACKGROUND

Atrial Fibrillation (AF) is the most common cardiac arrhythmia, affecting millions worldwide and contributing significantly to morbidity and mortality. Catheter ablation, particularly Pulmonary Vein Isolation (PVI), has become the standard of care for patients with symptomatic drug-refractory AF. The primary objective of PVI is to electrically isolate pulmonary veins, which are recognized as the primary sources of ectopic triggers for AF. Despite its widespread adoption, the efficacy of PVI is limited in cases of persistent AF, with recurrence rates approaching 50% after a single procedure. This limitation underscores the need to address the complex mechanisms underlying AF, including atrial remodeling, fibrosis, and non-pulmonary vein drivers such as localized rotors, micro-reentrant circuits, and regions of spatiotemporal dispersion ([1], [2]).

In patients with paroxysmal AF, PVI demonstrates higher efficacy, but its success diminishes in persistent AF cases, necessitating additional ablation strategies to target non-pulmonary vein drivers. Several adjunctive strategies have been explored to improve outcomes, including linear lesions based on the Cox maze procedure, which target the left atrium roof and mitral isthmus to prevent macro-reentrant atrial tachycardia. However, incomplete linear lesions often result in proarrhythmic effects. Similarly, targeting Complex Fractionated Atrial Electrograms (CFAEs), once thought to indicate AF drivers, showed no significant benefit over PVI alone in studies like STAR-AF II, leading to its decline in clinical use.

Other strategies, such as the stepwise approach combining linear ablation and CFAE defragmentation, have fallen out of favor due to poor long-term outcomes and high recurrence rates. Left Atrial Posterior Wall Isolation (LAPWI) demonstrated theoretical advantages but showed inconsistent results in trials like CAPLA, partly due to difficulties in achieving durable electrical isolation. Substrate ablation, targeting low-voltage areas to modify the arrhythmic substrate, has shown promise in specific populations, such as in the ERASE AF study, yet its effectiveness remains variable. Additional techniques, including Vein of Marshall ethanol infusion, have demonstrated incremental benefits in persistent AF, although further research is needed. Other approaches, such as ablation of non-pulmonary vein triggers and ganglionated plexi, present mixed results with concerns about safety and efficacy limiting their adoption.

Current ablation strategies are limited by overreliance on empirical methods, often necessitating extensive lesion sets. These methods increase the risk of tissue damage, proarrhythmic effects, or atrial stiffening without guaranteeing long-term success. Many strategies fail to account for the temporal stability of AF drivers, a key factor correlating with ablation success ([3], [4]). Consequently, ablation of AF drivers is frequently nonspecific and overly extensive, leading to limited improvements in outcomes and higher complication rates.

Advancements in mapping technologies have aimed to improve the precision of AF driver identification. Technologies such as FIRM (Focal Impulse and Rotor Modulation) [5], the iAMiFM algorithm [6], and Artificial Intelligence (AI)-based detection methods [7] focus on identifying and ablating reentrant sources and regions of spatiotemporal dispersion. While these innovations offer potential, their clinical success remains variable due to technical challenges, complex interpretations, and reliance on proprietary algorithms. Empirical evidence suggests that targeting regions with complex electrograms, low-voltage areas, and spatiotemporal dispersion may improve ablation outcomes [8], but these approaches remain inconsistent without a mechanistic understanding of AF maintenance [9].

A significant challenge in AF ablation lies in the lack of personalized treatment planning. Most procedures adopt a one-size-fits-all approach, neglecting unique anatomical and electrophysiological characteristics of individual patients. Personalized computational models, or cardiac digital twins, provide a promising solution by simulating patient-specific AF dynamics and predicting the outcomes of targeted ablation strategies. Despite their potential, these technologies face challenges in clinical adoption due to the complexity of creating and validating accurate, personalized models [10].

Therefore, there is a need of a system and method that improves the precision of AF driver identification, to improve AF termination rates and reducing recurrence after ablation of the identified AF drivers.

## References

[1] Ríos-Muñoz, G. R. et al., "Structural Remodeling and Rotational Activity in Persistent/Long-Lasting Atrial Fibrillation: Gender-Effect Differences and Impact on Post-Ablation Outcome", Frontiers in Cardiovascular Medicine, vol. 9, article 819429, March 2022.

[2] Rappel W. J. et al., "Spatially Conserved Spiral Wave Activity during Human Atrial Fibrillation", Circulation: Arrhythmia and Electrophysiology, vol. 17, p. E012041, March 2024.

[3] Honarbakhsh S. et al., "A Novel Mapping System for Panoramic Mapping of the LeftAtrium: Application to Detect and Characterize Localized Sources Maintaining AF", JACC: Clinical Electrophysiology, November 2017.

[4] Honarbakhsh S. et al., "Characterization of drivers maintaining atrial fibrillation: Correlation with markers of rapidity and organization on spectral analysis", Heart Rhythm, vol. 15, pp. 1296-1303, September 2018.

[5] Narayan S. M. et al., "Computational Mapping Identifies Localized Mechanisms for Ablation of Atrial Fibrillation", PLoS ONE, vol. 7, no. 9, p. e46034, 2012.

[6] Quintanilla J. G. et. al, "Instantaneous Amplitude and Frequency Modulations Detect the Footprint of Rotational Activity and Reveal Stable Driver Regions as Targets for Persistent Atrial Fibrillation Ablation", Circulation Research, vol. 125, pp. 609-627, August 2019.

[7] Seitz J. et al., "Artificial intelligence-adjudicated spatiotemporal dispersion: A patient-unique fingerprint of persistent atrial fibrillation", Heart Rhythm, January 2024.

[8] Franco E. et al., "Subjective identification and ablation of drivers improves rhythm control in patients with persistent atrial fibrillation. The CHAOS-AF study", Revista española de cardiologia (English ed.), 2 2024.

[9] Benali K. et al., "Recurrences of Atrial Fibrillation Despite Durable Pulmonary Vein Isolation: The PARTY-PVI Study", Circulation: Arrhythmia and Electrophysiology, vol. 16, p. E011354, March 2023.

[10] Roney C. H. at al., "Constructing bilayer and volumetric atrial models at scale", Interface Focus, vol. 13, December 2023.

[11] Jaffery O. A. et al., "A Review of Personalised Cardiac Computational Modelling Using Electroanatomical Mapping Data", Arrhythmia and Electrophysiology Review, vol. 13, 2024.

[12] Ríos-Muñoz G. R. et al., "Real-Time Rotational Activity Detection in Atrial Fibrillation", Frontiers in Physiology, vol. 9, p. 208, March 2018.

[13] Ríos-Muñoz G. R. et al., "Real-Time Ventricular Cancellation in Unipolar Atrial Fibrillation Electrograms", Frontiers in Bioengineering and Biotechnology, vol. 8, September 2020.

[14] Schilling C. et al., "Non-Linear Energy Operator for the Analysis of Intracardial Electrograms", 2009.

[15] Ríos-Muñoz G. R. et al., "Hidden Markov Models for Activity Detection in Atrial Fibrillation Electrograms," Computing in Cardiology Conference (CinC), pp. 1-4, December 2020

## SUMMARY

The present invention refers to a system and a computer-implemented method for detecting AF drivers in signals that exhibit automatic auto-accelerated activity.

The invention uses an "atrial electrogram entanglement" concept in multi-electrode intracavitary electrograms (EGMs), focusing on the spatiotemporal characteristics of EGM patterns, and identifying regions of accelerated and entangled activations as potential AF drivers. The invention detects this entanglement by calculating an entanglement index, which is used for identifying AF drivers in Electro-Anatomical Mapping (EAM) studies. The detection of AF drivers may be subsequently used in a targeted ablation of the entanglement regions, e.g., a radial ablation strategy, which improves AF termination rates compared to standard PVI alone, enhancing the precision of AF ablation, reducing recurrence, and ultimately improving patient outcomes.

The invention may be integrated with cardiac digital twins to personalize AF detection and ablation strategies using patient-specific anatomical and electrophysiological data, since digital twin technology enables simulation of AF propagation and evaluation of driver ablation impact in a controlled in silico environment, facilitating validation and refinement of this technique before clinical application [11]. Multiple simulations were performed in the digital twin, proving that the detected entanglement is due to reentrant mechanisms, thus linking entanglement detection to AF drivers detection.

The system for detecting atrial fibrillation drivers comprises a detection unit with a memory having programs or instructions storing therein and a data processing unit, such as a processor, configured to perform the following actions when executing the instructions:

- Receiving at least one set of unipolar atrial electrograms (EGMs), which may be stored in a data storage unit or acquired in real-time from a subject's heart, each set of unipolar EGMs comprising at least three unipolar EGMs corresponding to a multi-electrode catheter acquisition.

- Obtaining a set of bipolar EGMs for each set of unipolar EGMs.

- Calculating cycle lengths in each bipolar EGM.

- Detecting activity intervals in each bipolar EGM.

- Classifying the activity intervals of each bipolar EGM into discrete intervals and high-frequency complex activity (HFCA) intervals, depending on the duration of the activity interval. When the duration is lower than an activity threshold, the activity interval is classified as a discrete interval; otherwise, it is classified as an HFCA interval.

- Calculating an entanglement index for each HFCA interval, wherein the entanglement index depends on the detection, within an entanglement time window that at least partially includes the HFCA interval, of one or more discrete intervals in other bipolar EGMs of the set of bipolar EGMs with a reduction in the cycle length during the HFCA interval.

- Detecting atrial fibrillation drivers in each multi-electrode catheter acquisition based on the entanglement indexes.

In an embodiment, the data processing unit is configured to measure the reduction in the cycle length by comparing the cycle lengths associated to the discrete intervals contained in a current time window that at least partially includes the HFCA interval with the cycle lengths associated to the discrete intervals contained in a previous time window and/or in a subsequent time window.

In an embodiment, the activity threshold is independent for each bipolar EGM, and the data processing unit is configured to calculate the value of the activity threshold for each bipolar EGM using the median of the cycle lengths of the corresponding bipolar EGM comprised within a time window. A predetermined percentage of the median is preferably used.

The data processing unit may be further configured to generate and output an entanglement density map including the entanglement indexes obtained for each multi-electrode catheter acquisition and an associated 3D position.

The data processing unit may be configured to determine the cycle lengths of each bipolar EGM by detecting local activation times (LATs) on the corresponding unipolar EGMs.

The system may further comprise an amplifier configured to amplify at least one set of unipolar intracavitary EGMs from a multi-electrode catheter in contact with the heart of a subject, a filter configured to filter the at least one set of amplified intracavitary EGMs, and an ADC configured to convert the at least one set of filtered intracavitary EGMs into the at least one set of unipolar EGMs. The system may also include the multi-electrode catheter.

In an embodiment, the system also comprises a 3D mapping system configured to obtain a 3D position for each multi-electrode catheter acquisition.

Another aspect of the present invention refers to a computer-implemented method of detecting atrial fibrillation drivers, the method comprising the steps executed by the data processing unit already described.

A further aspect of the present invention refers to a non-transitory computer-readable storage medium having instructions stored thereon that, when executed by one or more processors, cause the one or more processors to perform the method previously described.

## BRIEF DESCRIPTION OF THE DRAWINGS

A series of drawings which aid in better understanding the invention and which are expressly related to an embodiment of said invention, presented as a non-limiting example thereof, are very briefly described below.

Figures 1A and 1B show an embodiment of a system for offline detection of AF drivers.

Figure 2 depicts another embodiment of the system that can be used for real-time AF driver analysis on a subject.

Figure 3 is a flow diagram of a method for offline detection of AF drivers, by analyzing EGM data stored in a storage unit.

Figures 4A and 4B show the effect of a rotor circulating through the electrodes of a multi-electrode catheter.

Figure 5 depicts the bipolar LATs detected in a bipolar EGM.

Figure 6 shows the activity intervals detected in a bipolar EGM.

Figure 7 depicts the classification of the activity intervals of Figure 6 into discrete intervals and HFCA intervals.

Figure 8 represents the activity intervals of a set of bipolar EGMs acquired by a multi-electrode catheter.

Figure 9 shows the bipolar EGMs in a PentaRay® catheter considered in Figured 8.

Figure 10 shows the computing of the entanglement index for an HFCA interval contained within the entanglement search window depicted in Figure 8.

Figure 11 depicts the entanglement indexes obtained for the set of bipolar EGMs of Figure 8.

Figure 12 shows the activity intervals classification for a multi-electrode catheter acquisition in which all the activity intervals are discrete intervals.

Figure 13 depicts the entanglement index graph corresponding to the example of Figure 12.

Figure 14 shows an exemplary entanglement density map obtained by the system.

## DETAILED DESCRIPTION

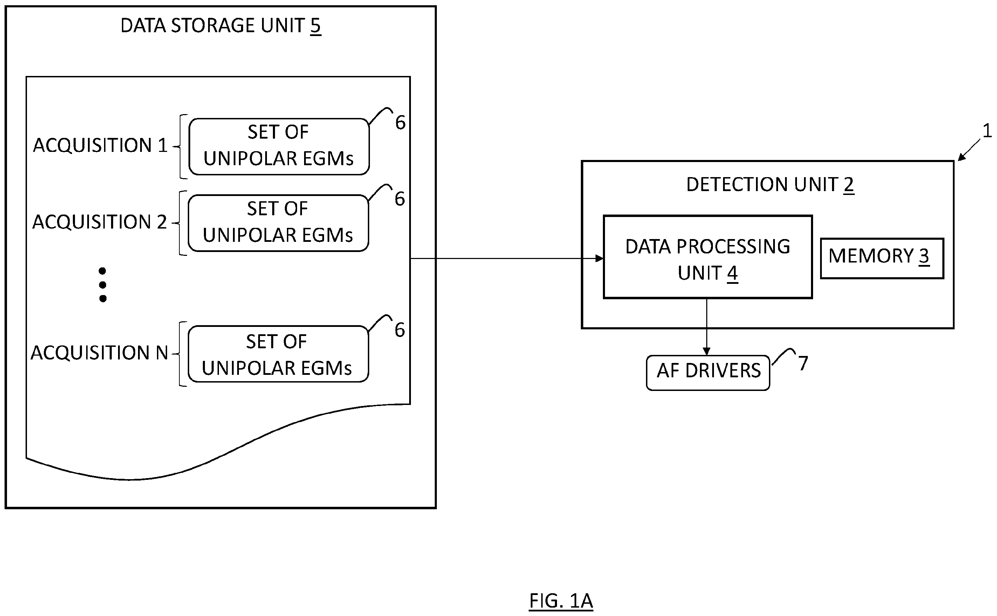

**Figure 1A** shows an embodiment of a system 1 for detecting atrial fibrillation (AF) drivers. The system 1 comprises a detection unit 2 with a memory 3 and a data processing unit 4 that retrieves at least one set 6 of unipolar atrial electrograms (EGMs) stored in a data storage unit 5 (e.g., a hard drive), each set 6 of unipolar EGMs comprising at least three unipolar EGMs corresponding to a different acquisition (acquisition 1, acquisition 2,..., acquisition N) from a multi-electrode catheter.

The data processing unit 4 is configured to obtain a set of bipolar EGMs for each set 6 of unipolar EGMs (each set of bipolar EGMs comprising at least two bipolar EGMs) and detect AF drivers 7 by analyzing the sets of bipolar EGMs. In this embodiment, the data processing unit 4 obtains the set of bipolar EGMs by subtracting, and subsequently filtering, pairs of unipolar EGMs of the set 6 of unipolar EGMs. This analysis is performed offline, after the sets 6 of unipolar EGMs have been captured and stored in the data storage unit 5 (e.g., a day after the acquisition).

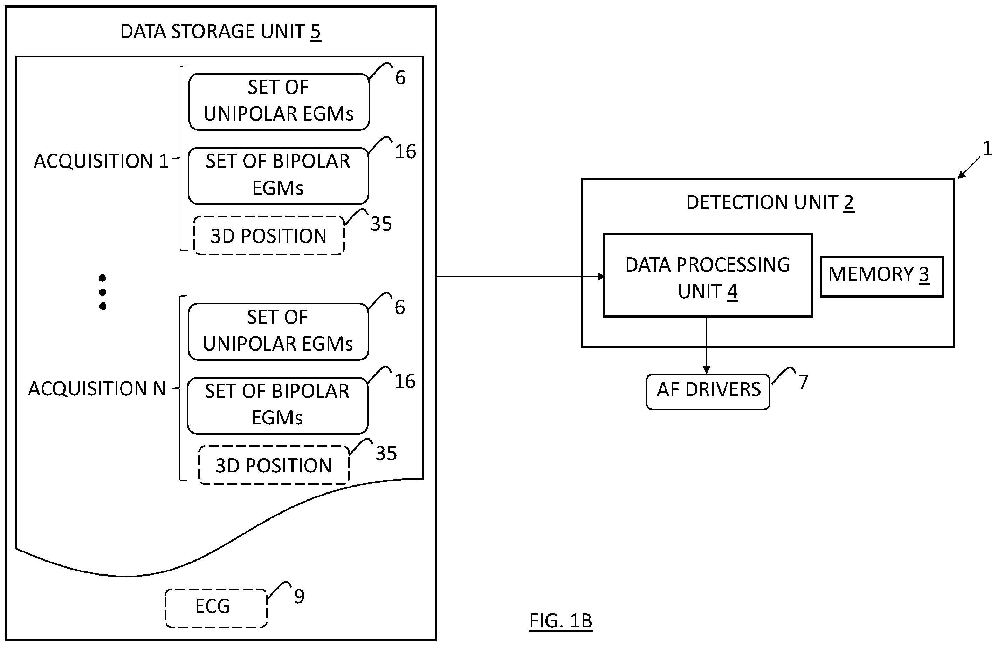

**Figure 1B** depicts another embodiment, in which the set 16 of bipolar EGMs have been previously obtained by an external system and stored in the data storage unit 5. In this case, the data processing unit 4 is configured to retrieve both the sets 6 of unipolar EGMs and the sets 16 of bipolar EGMs. In this embodiment, and also in the embodiment of Figure 1A, the data storage unit 5 may further include an electrocardiogram (ECG 9), so that the data processing unit 4 can also access the data storage unit 5 to retrieve the ECG 9 for far-field ventricle cancellation to recover hidden atrial activations in the unipolar EGMs [13]. Each acquisition may also include a corresponding associated 3D position 35 obtained by a navigation or 3D mapping system. All the optional signals stored in the data storage unit 5 are depicted with dashed lines in this embodiment.

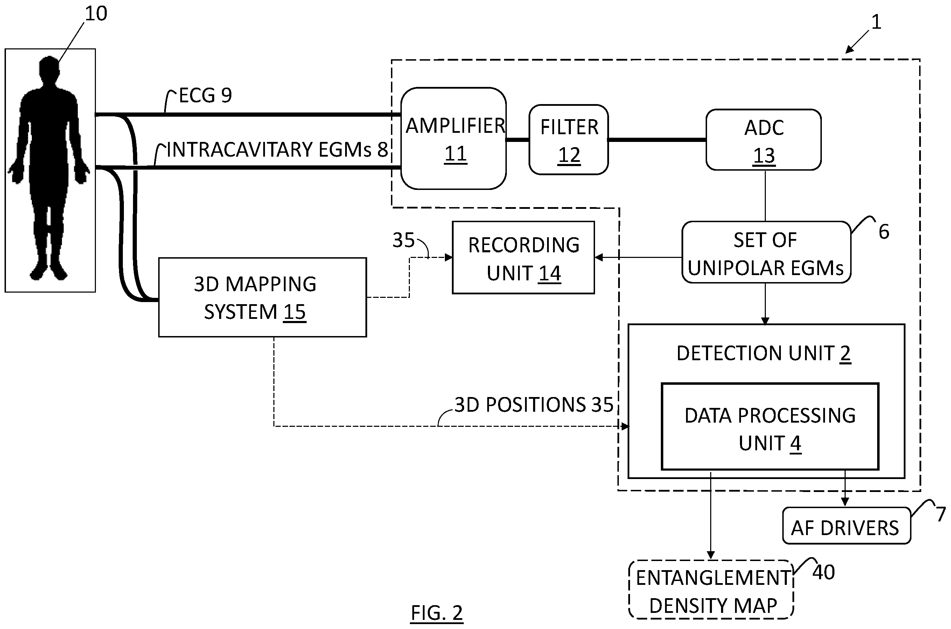

**Figure 2** depicts another embodiment in which the analysis is performed in real-time, at the same time intracavitary EGMs 8 are being acquired from a subject 10. In this embodiment, the system 1 comprises an amplifier 11, which receives unipolar intracavitary EGMs 8, generated by a multi-electrode catheter in contact with the heart of the subject 10, and a reference signal comprising an ECG 9 produced by another set of electrodes (e.g., ten electrodes in contact with pre-established external points of the subject's body, four of them being at peripheral points and six of them at precordial points). The intracavitary EGMs 8 represent the electrical activity of the chambers of the heart, whereas the ECG 9 is used as a reference signal for synchronization and elimination of unwanted noise in the intracavitary EGMs 8. The system 1 also comprises a filter 12 that eliminates the low and high-frequency noise produced by the electrodes, applying a filter (normally, a 0-100Hz low-pass filter for the unipolar signals and, if the filter also receives amplified bipolar EGMs, a 30-240Hz band-pass filter for the bipolar signals). A band-stop Notch filter may also be applied to cancel power line interference (50Hz/60Hz).

In this embodiment, the system 1 also includes an analog-to-digital converter, ADC 13, which converts the intracavitary EGMs 8 to digital data, obtaining the sets 6 of unipolar EGMs for different acquisitions and which may be stored by a recording unit 14 (which may be external to the system) in a data storage unit 5 for offline analysis (e.g. as in the embodiment of Figure 1A), optionally together with the sets 16 of bipolar EGMs and/or the ECG 9 (as in Figure 1B). A 3D mapping system 15 may obtain 3D positions 35 during the signals acquisition to generate a 3D map that reconstructs the anatomy of the heart of the subject 10. The 3D positions 35 acquired during the multi-electrode catheter acquisitions may be reported to the detection unit 2 and/or the recording unit 14 for storing in a memory or a data storage unit, such that each acquisition is associated to a particular 3D position.

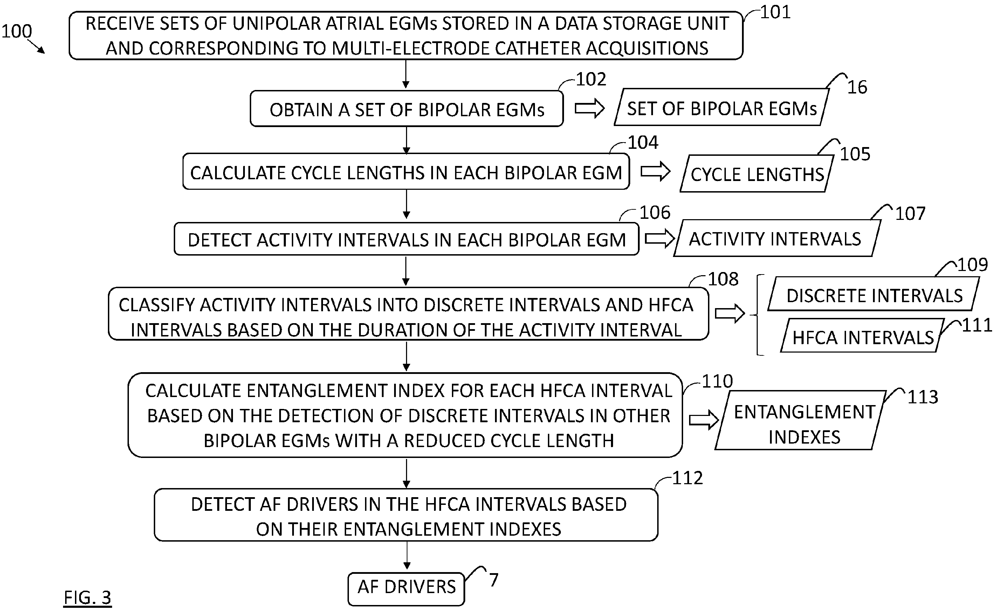

The retrieved sets 6 of unipolar EGMs, for different multi-electrode acquisition locations within an atrium of the subject's heart, are analyzed in real-time by the detection unit 2, following the process depicted in **Figure 3,** to detect AF drivers 7 in the atrium. The identification of AF drivers 7 can be later used in an ablation process to eliminate the negative effect caused by the detected AF drivers. Figure 3 is a flow diagram of a computer-implemented method 100 of detecting AF drivers according to an embodiment. The method 100 comprises the following steps:

- Receiving 101 at least one set 6 of unipolar EGMs, stored in a data storage unit 5, each set 6 of unipolar EGMs comprising at least three unipolar EGMs and corresponding to a multi-electrode catheter acquisition.

- Obtaining 102 a set 16 of bipolar EGMs for each set 6 of unipolar EGMs. Each set 16 of bipolar EGMs comprises at least two bipolar EGMs. The sets 16 of bipolar EGMs may be obtained by the data processing unit 4 directly from the sets 6 of unipolar EGMs (by subtracting pairs of unipolar signals) or, if stored in the data storage unit 5 (Figure 1B), by retrieving the sets 16 of bipolar EGMs stored therein.

- Calculating 104 cycle lengths 105 in each bipolar EGM of the at least one set 16 of bipolar EGMs.

- Detect activity intervals 107 in each bipolar EGM of the at least one set 16 of bipolar EGMs. This step 106 and the previous step 104 can be performed in any order or even in parallel.

- Classify 108 the activity intervals 107 of each bipolar EGM into discrete intervals 109, when the duration of the activity interval is lower than an activity threshold, and high-frequency complex activity intervals (HFCA intervals 111), when the duration of the activity interval is higher than the activity threshold.

- Calculate 110 an entanglement index 113 for each HFCA interval 111, wherein the entanglement index 113 depends on the detection, within an entanglement time window that at least partially includes the HFCA interval 111, of one or more discrete intervals 109 in other bipolar EGMs of the set 16 of bipolar EGMs with a reduction in the cycle length 105 during the HFCA interval.

- Detect 112 AF drivers 7 in each multi-electrode catheter acquisition based on the calculated entanglement indexes 113.

The method is compatible with any catheter model, electrode layout or intracavitary deployment, provided that the multi-electrode catheter acquires at least two bipolar EGMs.

The entanglement index 113 is used for detecting AF drivers 7. The entanglement index 113 measures the temporal behaviour of accelerated EGMs from neighbouring electrodes within a specific time window, serving as a marker for AF drivers. The present invention detects EGM acceleration produced in one or more discrete intervals 109 in the vicinity of an interval of high-frequency complex, fragmented activity (HFCA interval 111).

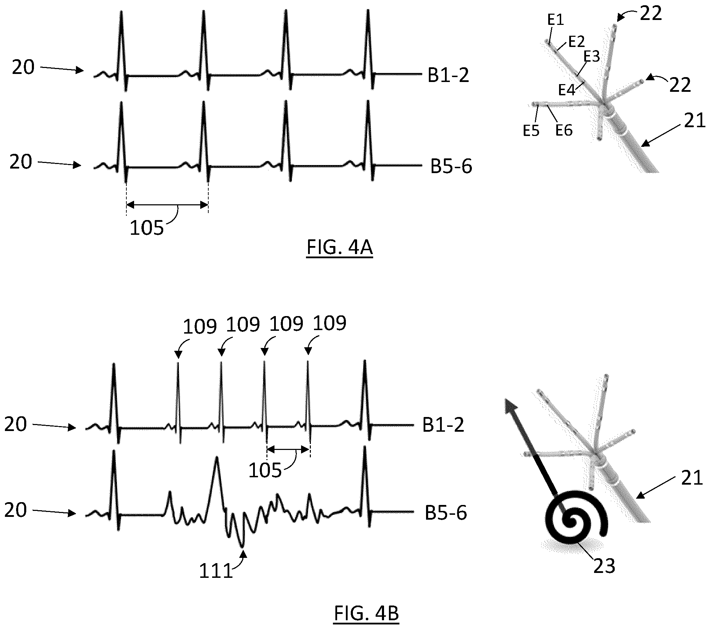

**Figures 4A** and **4B** show the bipolar EGM 20 (bipolar B1-2) between electrodes E1 and E2 of a multi-electrode catheter 21, such as a PentaRay® (composed of 20 electrodes arranged in five radial splines 22), and the bipolar EGM 20 (bipolar B5-6) between electrodes E5 and E6. An AF driver 7 located at the acquisition location of the multi-electrode catheter 21 temporarily generates circular and rotational activation patterns, known as rotors 23, that pass through the electrodes. In Figure 4A a stable condition is depicted (no rotors and the bipolar EGMs 20 are stable, all the activity intervals 107 are discrete intervals 109), whereas in Figure 4B a rotor 23 is passing through electrodes E5 and E6, which causes a high-frequency complex activity (HFCA interval 111) in bipolar B5-6 and the cycle lengths 105 of the discrete intervals 109 in bipolar B1-2 are shortened. This EGM acceleration in the temporal vicinity of an interval of complex, fragmented activity, shown in Figure 4B, allows AF drivers 7 detection.

The calculation of the cycle lengths 105 in the bipolar EGMs 20 may be performed according to any method known in the prior art. In an embodiment, the EGM cycle lengths 105 are determined by detecting bipolar LATs (local activation times) in the bipolar EGMs 20, using the unipolar LAT detections as explained for instance in [12] and patent document WO2019063861-A1. The cycle lengths of the EGM signals can be calculated as the difference between consecutive unipolar LATs. For the AF unipolar signal, a ventricular far-field cancellation is preferably applied [13] using the ECG 9, if available, to obtain more precise LATs detection. Each unipolar activation is detected using an algorithm that enhances the voltage signal's slope prior to identifying its maximum negative slope, employing an adaptive exponential decay threshold [12]. This adaptive filter enables interval detection despite variations in signal magnitude caused by unstable catheter positioning, electrode contact, or fluctuations in EGM amplitude, particularly pronounced in AF [13].

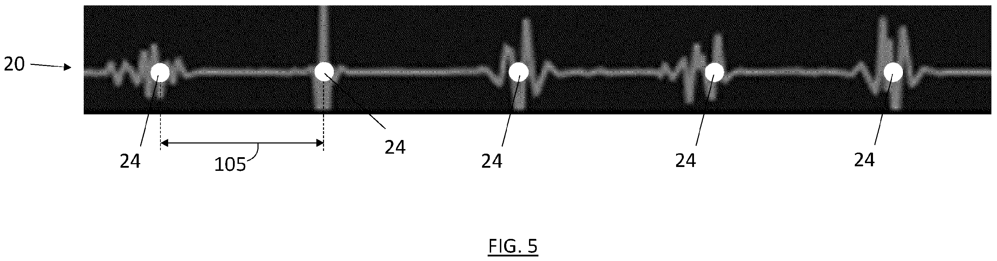

In an embodiment, the LATs detection is performed twice: once on the original unipolar signals and again on their mirrored counterparts. The LATs from both signals are compared to correct any discrepancies and then averaged the results to assign the unipolar LATs to the bipolar EGMs. Finally, any LAT activations with no bipolar EGM signal energy are discarded, using for instance a bipolar NLEO activity binary signal [14] as an extra filter to eliminate any LATs falling outside the bipolar energy window. **Figure 5** shows the bipolar LATs 24 detected in a bipolar EGM 20 and the cycle lengths 105 determined between consecutive bipolar LATs 24.

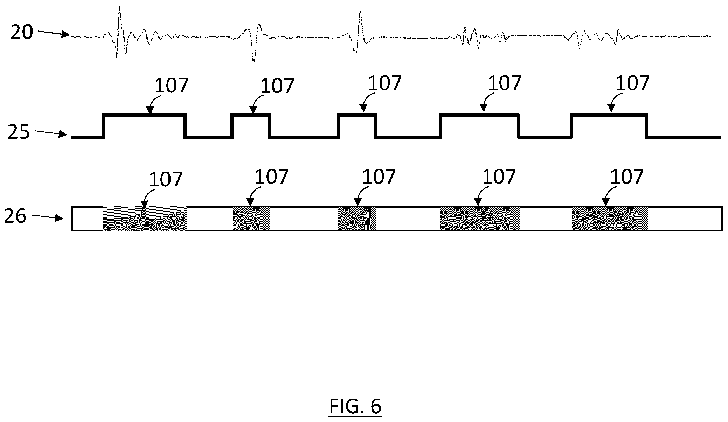

**Figure** 6 depicts the detection of activity intervals 107 in a bipolar EGM 20. Any method known in the prior art may be used to that end. In an embodiment, an EGM energy detection threshold, which detects activity from no signal (flat zero isoelectrical line), is applied to each bipolar EGM 20. The bipolar EGMs 20 may be processed to detect activity intervals 107 of significant activity in them by creating activity windows over them with an energy operator. In an embodiment, the binary activity function is obtained using the non-linear energy operator (NLEO) developed in [14], although other energy detection methods may be used. The method approximates the signal to a mass-spring system whose energy is computed and classified. An adaptive thresholding method determines if the intervals of the signal are considered to be active or inactive. In an embodiment, the parameters are set according to a previous study [15] in which activity detection in bipolar EGMs was validated and optimized with expert annotations. The resulting classification outcome consists of a binary activity signal 25 displayed under the bipolar EGM 20, which is 0 at the intervals of negligible activity and 1 at the intervals of significant activity. Signal 26 depicts the binary activity signal 25 in a code color.

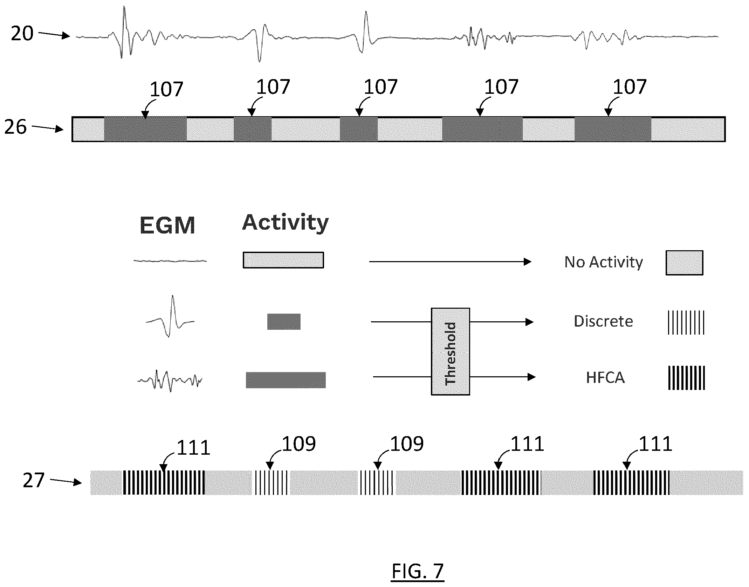

**Figure** 7 depicts the classification of the activity intervals 107 of the example of Figure 6 into discrete intervals 109 and HFCA intervals 111 based on the duration of the activity interval 107. The binary activity signal 25 (or the corresponding signal 26 depicted in code color) is classified with respect to the length of the activity intervals 107, labeling the ones below an activity threshold as discrete intervals 109 and those above the activity threshold as HFCA intervals 111, obtaining a classified signal 27 (the activity intervals 107 having a duration equal to the activity threshold may be classified as either discrete or HFCA intervals). This is based on the fact that discrete EGMs present a distinct shape with a moderate duration, whereas the fragmented signals, potentially affected by driver activity and atrial remodeling, present a lengthier chaotic wavy profile. In an embodiment, the activity threshold value for each bipolar EGM 20 depends on the median of the cycle lengths 105 of said bipolar EGM 20 comprised within a time window (the whole signal may be considered). A predetermined percentage of the median cycle length of the EGM is preferably used to detect an interval as discrete or HFCA.

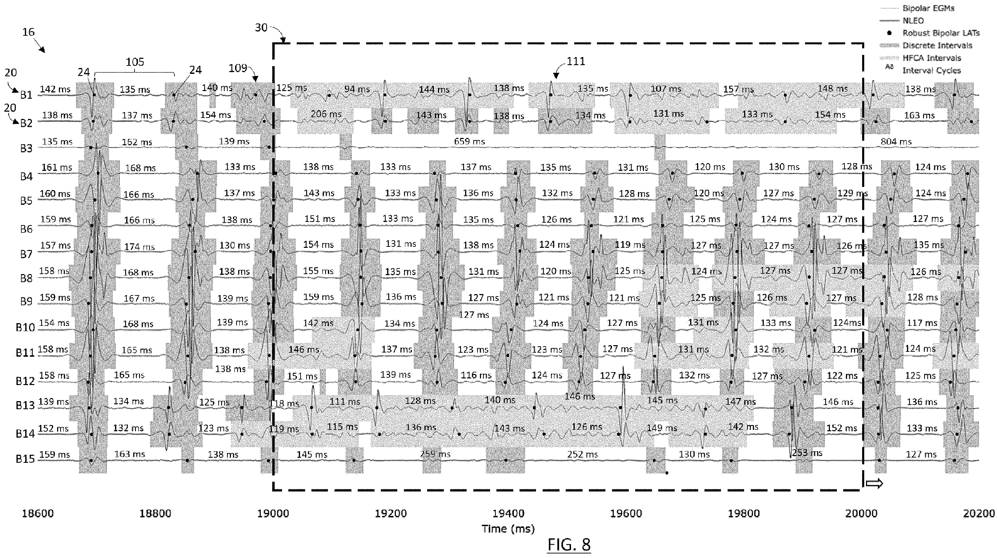

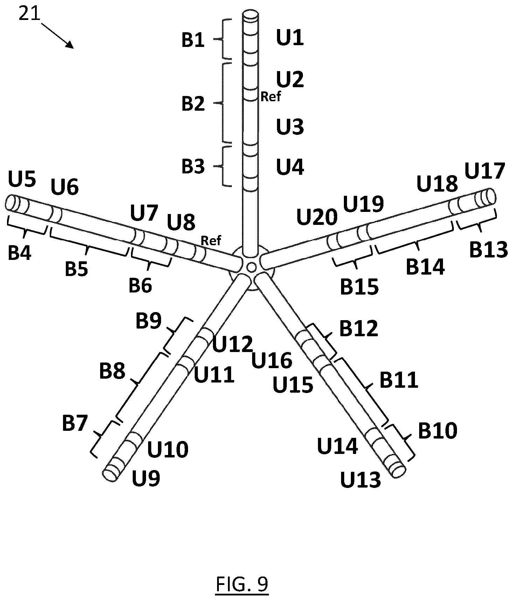

**Figure 8** depicts the activity intervals classification (discrete intervals 109 and HFCA intervals 111) in a set 16 of bipolar EGMs 20 acquired by a multi-electrode catheter 21, in particular a PentaRay® catheter, formed by 15 bipolar EGMs (or bipolar leads). With each acquisition of the PentaRay® catheter, 20 unipolar EGMs (U1, U2,..., U20) and 15 bipolar EGMs (B1, B2,..., B15) are obtained, as depicted in **Figure 9,** wherein B1=U2-U1, B2=U3-U2,..., B15=U20-U19. Figure 8 also shows the cycle lengths 105 and the bipolar LATs 24 obtained for each bipolar EGM 20.

Once the activity intervals 107 are classified, an entanglement index 113 is calculated for each HFCA interval 111. A condition for the calculation of the entanglement index 113 may be applied to the HFCA interval 111; for example, a minimum duration of the HFCA interval 111 (e.g., 250ms) may be required. Using the filtered bipolar EGMs 20 and classified intervals (discrete intervals 109 and HFCA intervals 111) from previous steps, the detection unit 2 inspects the 15 bipolar leads (B1, B2,..., B15) with a moving temporal window (entanglement search window 30) to detect HFCA intervals 111 and assign them entanglement values. The entanglement search window 30 used for entanglement detection is preferably a sliding window with a predetermined overlap. The analysis is preferably focused on short entanglement search windows 30, around 1 or 2 seconds long, so that local dynamics can be captured anytime. The entanglement search windows 30 should preferably be large enough in terms of atrial fibrillation to capture at least 3 consecutive activations. In an embodiment, the length of the entanglement search window 30 is comprised within [500ms - 1500ms], and the entanglement window overlap that determines the overlap between consecutive entanglement search windows 30 is comprised within [40% - 60%] of the size of the entanglement search window 30. In the embodiment depicted in Figure 8, the length of the entanglement search window 30 is 1000ms and the entanglement window overlap is 50%. Within this entanglement search window 30, intervals labeled as HFCA intervals 111 are analyzed to assess interval (cycle length) acceleration in the remaining leads containing discrete intervals 109 during the corresponding HFCA interval 111. Acceleration is measured as a reduction in the time intervals between discrete LATs on those leads.

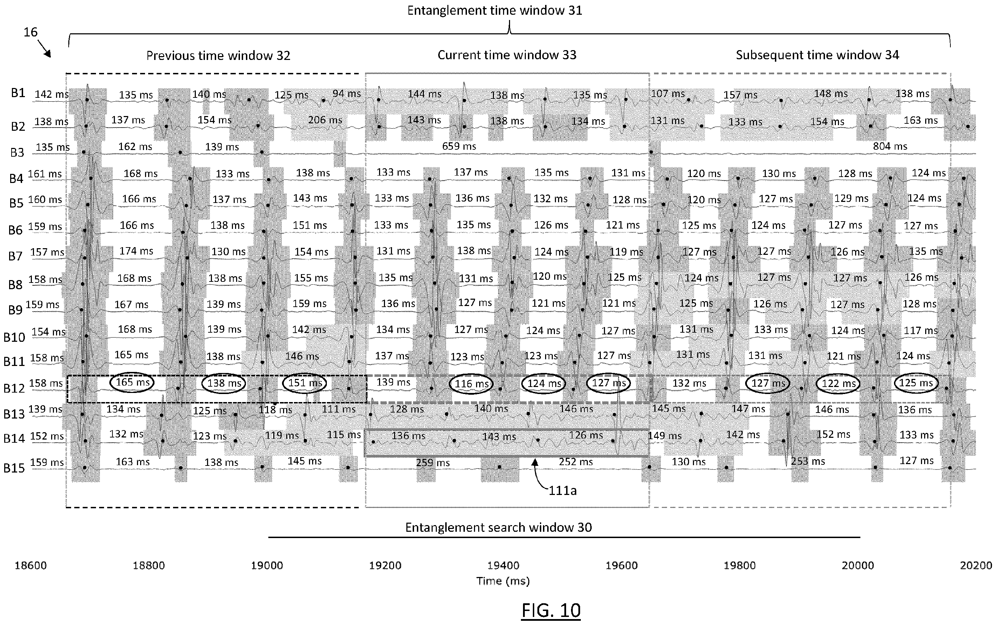

**Figure 10** shows an exemplary embodiment for computing the entanglement index 113 for the HFCA intervals 111 contained within the entanglement search window 30 depicted in Figure 8. In an embodiment, an Electrogram Activity Index (EAI) is calculated for each bipolar EGM 20 and for the entanglement search window 30 (or another time window). The EAI will be later employed to calculate the entanglement index 113. For each bipolar EGM 20, a Discrete Index (DI) is calculated for the discrete intervals 109 as the total length in time of the discrete intervals 109 contained within the entanglement search window 30 and divided by the length of the entanglement search window 30, and for the HFCA intervals 111 a similar HFCA Index (HFCAI) is also obtained (i.e. the total length in time of the HFCA intervals 111 contained within the entanglement search window 30 divided by the length of the entanglement search window 30). Then, the EAI for each bipolar EGM 20 may be obtained for the corresponding entanglement search window 30 as:

$$EAI = \frac{DI - HFCAI}{DI + HFCAI}$$

The range of the EAI is [-1, 1], where a value of +1 will describe a bipolar EGM 20 consisting only of discrete activity (i.e. discrete intervals 109) during the entanglement search window 30 and a value of -1 would be for completely fragmented continuous activity (i.e. only HFCA intervals 111) during the entanglement search window 30. Any intermediate signals ($-1 \leq EAI \leq 1$) are defined as hybrid, e.g., $EAI = 0$ would imply 50% discrete and 50% HFCA mixed EGM contributions.

As previously explained, for each HFCA interval 111 detected in the entanglement search window 30, an analysis is carried out to detect, within an entanglement time window 31 that includes the HFCA interval 111, a reduction in the duration of discrete intervals 109 of the other bipolar EGMs (i.e. the bipolar EGMs 20 different from the one containing the corresponding HFCA interval 11 under analysis). Any acceleration detected in any other lead, based on their discrete LAT annotations, contributes to an increase in the entanglement value. The classification of leads within the entanglement search window 30 (or within another time window, such as the entanglement time window 31) based on their EAI -whether discrete, fragmented or hybrid- can be used to calculate the entanglement index 113. In an embodiment, for purely discrete leads ($EAI = 1$) with detected acceleration, the entanglement value is increased by 1 unit, whereas for hybrid leads ($-1 \leq EAI \leq 1$) with discrete acceleration the entanglement value is increased by 0.5 units. Leads with only HFCA intervals 111 (i.e. completely fragmented continuous activity, $EAI = -1$ ) are excluded from analysis due to the unreliability of multiple activations and deflections in unipolar EGMs.

An exemplary calculation of the entanglement index 113 for HFCA interval 111a in bipolar B14 of Figure 10 will be now explained. The reduction in the cycle lengths 105 in another bipolar EGM during the HFCA interval 111a is measured by comparing the discrete cycle lengths (i.e. cycle lengths containing discrete intervals 109) of said another bipolar EGM 20 contained in a current time window 33 that at least partially includes said HFCA interval 111a with the discrete cycle lengths contained in the entanglement time window 31. In an embodiment, the discrete cycle lengths of the current time window 33 are compared with the discrete cycle lengths contained in a time window prior to the current time window (previous time window 32) and/or following the current time window (subsequent time window 34). In an embodiment, discrete cycle lengths having a duration longer than a maximum threshold (such as 300 ms) are not included in the analysis, since they would be very slow to be atrial fibrillation. In the example, the size of the previous time window 32 and the subsequent time window 34 have a configurable, predetermined length (in the example of Figure 10 their lengths are 500ms).

In the example:

- The complete discrete cycle lengths (only including discrete intervals 109) in bipolar B12 during the current time window 33 have the following duration: 116ms, 124ms, 127ms (the cycle length of 139ms is not completely included in the current time window 33 and is thus disregarded). An average value for the current time window 33 is obtained, preferably the median (median=124ms) to discard outliers, although a mean value (mean=122.33ms) may also be considered.

- For the previous time window 32, the complete discrete cycle lengths have a duration of 165ms, 138ms and 151ms, the median being 151ms (mean=151.3ms).

- For the subsequent time window 34, the complete discrete cycle lengths have a duration of 127ms, 122ms and 125ms, the median being 125ms (mean=124.6ms). The first cycle length of 132ms is not completely included in the subsequent time window 34 and is discarded.

Since the median of the discrete cycle lengths of the current time window 33 (124ms) is lower than the median of the discrete cycle lengths of the previous time window 32 (151ms), there is a reduction in the cycle lengths during the HFCA interval 111a in bipolar B12, and the entanglement index 113 is increased by 1 unit, since $EAI = 1$ for B12 during the entanglement search window 30. Besides, there is also a further reduction in the cycle lengths of the current time window (median=124ms) when compared to the subsequent time window 34 (median=125ms), and the entanglement index 113 is additionally increased by 1 unit. The contribution of B12 to the entanglement index 113 of HFCA interval 111a is 2 units. The other bipolar EGMs (B1-B11, B13 and B15) are also analyzed to determine whether there are additional contributions to the entanglement index 113 for HFCA interval 111a.

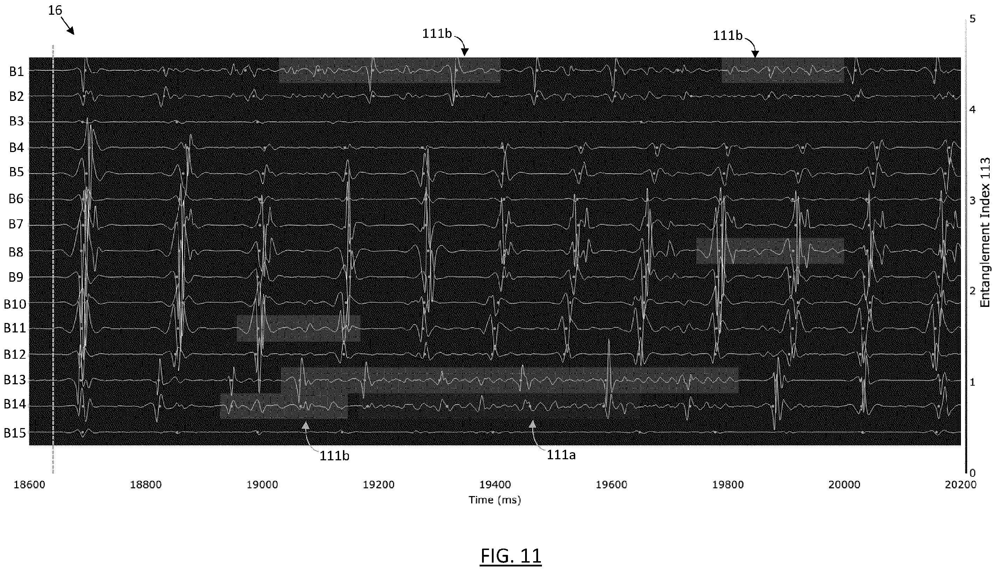

**Figure 11** depicts the entanglement index 113 (in color code) obtained according to the method described for all the HFCA intervals 111 of the set 16 of bipolar EGMs shown in Figure 8. This method prioritizes detecting discrete EGMs acceleration rather than fragmented activity and focuses on identifying the presence of acceleration rather than quantifying the extent of entanglement across multiple leads. The exemplary entanglement detection provided in Figure 11 highlights LATs acceleration and demonstrates the detection process for real AF signals.

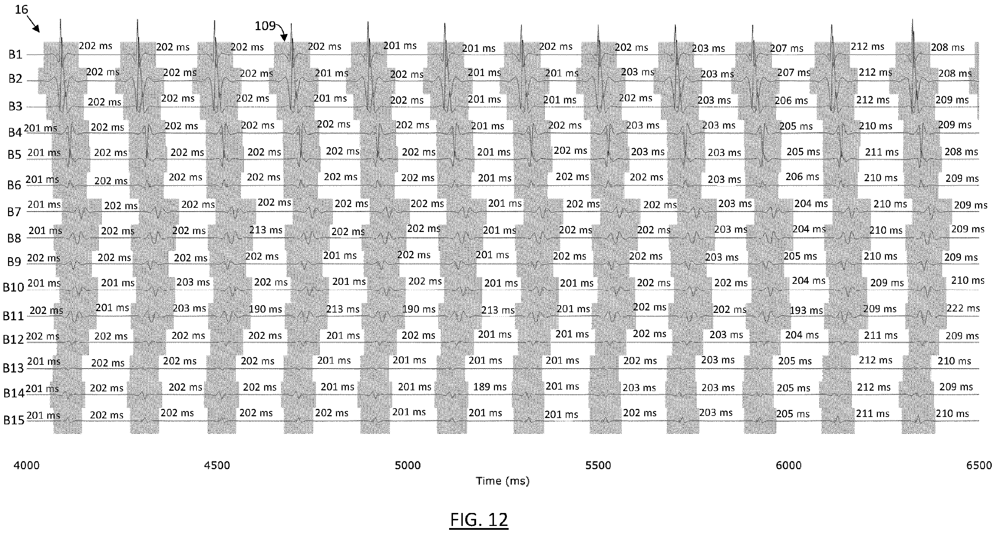

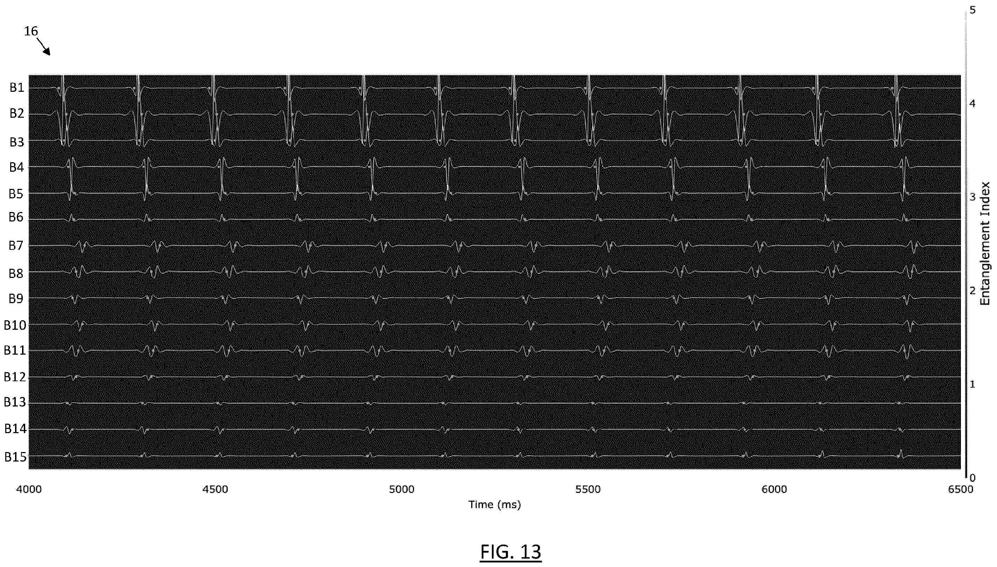

**Figure 12** shows the activity intervals classification for another multi-electrode catheter acquisition in which all the activity intervals 107 are discrete intervals 109 and therefore there is no entanglement. **Figure 13** depicts the corresponding entanglement index 113 graph wherein all the entanglement values are 0 since there is no HFCA interval.

Finally, AF drivers 7 are detected based on the entanglement index 113 of the HFCA intervals 111. In an embodiment, an AF driver is detected whenever the entanglement index 113 of at least a predefined number of HFCA intervals 111 are equal or greater (or just greater) than an entanglement threshold. In an embodiment, AF drivers 7 are detected when there is at least one HFCA interval 111 with an entanglement index 113 equal or greater than 1, although different conditions (predetermined number, entanglement threshold) may be considered. In the example depicted in Figure 11 the HFCA interval 111a has an entanglement index value of 2, and six other HFCA intervals 111b have an entanglement index value of 3; therefore, an AF driver 7 is detected for this multi-electrode catheter acquisition. However, no AF driver 7 is detected for the multi-electrode catheter acquisition corresponding to the set 16 of bipolar EGMs 20 of Figure 12, since the entanglement index 113 is always 0 (a default value for every sampling point of the bipolar EGMs 20 where there are no HFCA intervals 111), lower than the entanglement threshold (Figure 13).

The occurrence of atrial entanglement can be expressed, for instance, as a binary signal (0 for no entanglement, 1 when there is entanglement), or as a percentage of the time of the acquisition in which there is a detection of entanglement (the duration of the HFCA intervals 111 with entanglement index 113 equal or greater than 1 with regard to the total acquisition time).

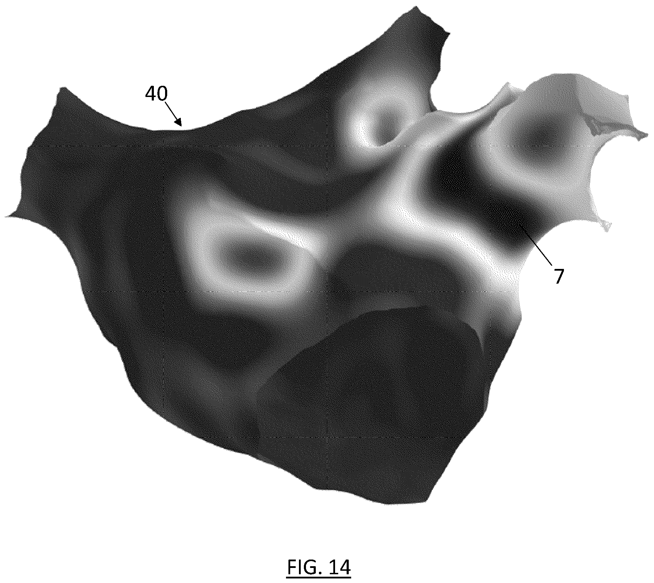

The detections of AF drivers 7 are associated to the multi-electrode acquisitions that have been analyzed. In addition, said acquisitions may be linked to 3D positions in the heart of the subject 10, obtained by the 3D mapping system 15 depicted in Figure 2. The 3D positions 35 associated to each acquisition (acquisition 1, acquisition 2,... , acquisition N) may be stored in the data storage unit 5 of Figure 1B (and retrieved by the data processing unit 4 during the entanglement analysis) or directly received from the 3D mapping system 15 and stored in the memory 3 of the detection unit 2. Using the 3D position 35 of each acquisition, the data processing unit 4 of the detection unit 2 may be further configured to generate and output (e.g., to a display, which may be part of the system 1) an entanglement density map 40 **(Figure 14)** showing in code color the entanglement indexes 113 obtained, and therefore the 3D positions of the AF drivers 7, in a reconstructed heart (or a part thereof) of the subject 10. The entanglement density map 40 may be used in an ablation process of the entanglement regions (the ones considered to include AF drivers 7).

If the detection unit 2 does not have access to the positions of the electrodes of the catheter captured by the 3D mapping system 15, the acquisitions are sequentially obtained and annotated, including data related to the existence or not of an AF driver 7 on each acquisition, such as the entanglement index 113 obtained for each acquisition. An external system, such as the 3D mapping system 15, having the 3D position data of the electrodes during the acquisitions will be able to correlate this data with the AF driver data (entanglement data), to generate for instance an entanglement density map 40 representative of occurrences of AF drivers in the heart of the subject 10.

**Claims**

1. A system for detecting atrial fibrillation drivers, the system (1) comprising a detection unit (2) with a memory (3) and a data processing unit (4) configured to:

   receive at least one set (6) of unipolar atrial electrograms, EGMs, each set (6) of unipolar EGMs comprising at least three unipolar EGMs corresponding to a multi-electrode catheter acquisition;
   obtain a set (16) of bipolar EGMs (20) for each set (6) of unipolar EGMs;
   calculate cycle lengths (105) in each bipolar EGM (20);
   detect activity intervals (107) in each bipolar EGM (20);
   classify the activity intervals (107) of each bipolar EGM (20) into discrete intervals (109), when the duration of the activity interval (107) is lower than an activity threshold, and high-frequency complex activity, HFCA, intervals (111), when the duration of the activity interval (107) is higher than the activity threshold;
   calculate an entanglement index (113) for each HFCA interval (111), wherein the entanglement index (113) depends on the detection, within an entanglement time window (31) that at least partially includes the HFCA interval (111), of one or more discrete intervals (109) in other bipolar EGMs (20) of the set (16) of bipolar EGMs (20) with a reduction in the cycle length (105) during the HFCA interval (111); and
   detect atrial fibrillation drivers (7) in each multi-electrode catheter acquisition based on the entanglement indexes (113).

2. The system of claim 1, wherein the data processing unit (4) is configured to measure the reduction in the cycle length (105) by comparing the cycle lengths (105) associated to the discrete intervals (109) contained in a current time window (33) that at least partially includes the HFCA interval (111) with the cycle lengths (105) associated to the discrete intervals (109) contained in a previous time window (32) and/or in a subsequent time window (34).

3. The system of any preceding claim, wherein the data processing unit (4) is configured to calculate the value of the activity threshold for each bipolar EGM (20) using the median of the cycle lengths (105) of the corresponding bipolar EGM (20) comprised within a time window.

4. The system of any preceding claim, wherein the data processing unit (4) is further configured to generate and output an entanglement density map (40) including the entanglement indexes (113) obtained for each multi-electrode catheter acquisition and an associated 3D position (35).

5. The system of any preceding claim, wherein the data processing unit (4) is configured to determine the cycle lengths (105) of each bipolar EGM (20) by detecting local activation times, LATs, on the corresponding unipolar EGMs.

6. The system of any preceding claim, further comprising:

   an amplifier (11) configured to amplify at least one set of unipolar intracavitary EGMs (8) from a multi-electrode catheter (21) in contact with the heart of a subject (10);
   a filter (12) configured to filter the at least one set of amplified intracavitary EGMs (8); and
   an ADC (13) configured to convert the at least one set of filtered intracavitary EGMs (8) into the at least one set (6) of unipolar EGMs.

7. The system of any preceding claim, further comprising a 3D mapping system (15) configured to obtain a 3D position (35) for each multi-electrode catheter acquisition.

8. A computer-implemented method of detecting atrial fibrillation drivers, the method (100) comprising:

   receiving (101) at least one set (6) of unipolar atrial electrograms, EGMs, stored in a data storage unit (5), each set (6) of unipolar EGMs comprising at least three unipolar EGMs corresponding to a multi-electrode catheter acquisition;
   obtaining (102) a set (16) of bipolar EGMs (20) for each set (6) of unipolar EGMs;
   calculating (104) cycle lengths (105) in each bipolar EGM (20);
   detecting (106) activity intervals (107) in each bipolar EGM (20);

classifying (108) the activity intervals (107) of each bipolar EGM (20) into discrete intervals (109), when the duration of the activity interval (107) is lower than an activity threshold, and high-frequency complex activity, HFCA, intervals (111), when the duration of the activity interval (107) is higher than the activity threshold; calculating (110) an entanglement index (113) for each HFCA interval (111), wherein the entanglement index (113) depends on the detection, within an entanglement time window (31) that at least partially includes the HFCA interval (111), of one or more discrete intervals (109) in other bipolar EGMs (20) of the set (16) of bipolar EGMs (20) with a reduction in the cycle length (105) during the HFCA interval (111); and detecting (112) atrial fibrillation drivers (7) each multi-electrode catheter acquisition based on the entanglement indexes (113).

9. The computer-implemented method of claim 1, wherein the reduction in the cycle length (105) is measured by comparing the cycle lengths (105) associated to the discrete intervals (109) contained in a current time window (33) that at least partially includes the HFCA interval (111) with the cycle lengths (105) associated to the discrete intervals (109) contained in a previous time window (32) and/or in a subsequent time window (34).

10. The computer-implemented method of any of claims 8 to 9, wherein the value of the activity threshold for each bipolar EGM (20) is calculated using the median of the cycle lengths (105) of the corresponding bipolar EGM (20) comprised within a time window.

11. The computer-implemented method of any of claims 8 to 10, further comprising generating an entanglement density map (40) including the entanglement indexes (113) obtained for each multi-electrode catheter acquisition and an associated 3D position (35).

12. The computer-implemented method of any of claims 8 to 11, wherein the cycle lengths (105) of each bipolar EGM (20) are determined by detecting local activation times, LATs, on the corresponding unipolar EGMs.

13. A computer program product, comprising instructions which, when the program is executed by a processor, cause the processor to carry out the method of any of claims 8 to 12.

14. A computer-readable storage medium comprising instructions which, when executed by a processor, cause the processor to carry out the method of any of claims 8 to 12.

FIG. 1A

FIG. 1B

DATA STORAGE UNIT 5

ACQUISITION 1
- SET OF UNIPOLAR EGMs — 6
- SET OF BIPOLAR EGMs — 16
- 3D POSITION — 35

ACQUISITION N
- SET OF UNIPOLAR EGMs — 6
- SET OF BIPOLAR EGMs — 16
- 3D POSITION — 35

ECG — 9

DETECTION UNIT 2 — 1
- DATA PROCESSING UNIT 4
- MEMORY 3

AF DRIVERS — 7

EP 4 763 088 A1

12

FIG. 2

FIG. 3

FIG. 4A

FIG. 4B

EP 4 763 088 A1

FIG. 5

FIG. 6

FIG. 7

FIG. 8

FIG. 9

FIG. 10

FIG. 11

FIG. 12

FIG. 13

FIG. 14

## EUROPEAN SEARCH REPORT

**Application Number**

EP 24 38 3410

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | GONZALO R. RÍOS-MUÑOZ ET AL: "Real-Time Rotational Activity Detection in Atrial Fibrillation", FRONTIERS IN PHYSIOLOGY, [Online] vol. 9, 13 March 2018 (2018-03-13), XP055613759, CH ISSN: 1664-042X, DOI: 10.3389/fphys.2018.00208 [retrieved on 2025-05-21] * abstract * | 1-14 | INV. A61B5/361 |
| A | US 2018/042503 A1 (CHAUHAN VIJAY SINGH [CA] ET AL) 15 February 2018 (2018-02-15) * abstract * * figures 3, 4 * | 1-14 | |
| A,D | RÍOS-MUÑOZ GONZALO R. ET AL: "Real-Time Ventricular Cancellation in Unipolar Atrial Fibrillation Electrograms", FRONTIERS IN BIOENGINEERING AND BIOTECHNOLOGY, [Online] vol. 8, 30 July 2020 (2020-07-30), page 789, XP055923698, DOI: 10.3389/fbioe.2020.00789 Retrieved from the Internet: URL:https://www.ncbi.nlm.nih.gov/pmc/artic les/PMC7406791/pdf/fbioe-08-00789.pdf> [retrieved on 2025-05-21] * abstract * | 1-14 | **TECHNICAL FIELDS SEARCHED (IPC)** A61B |

The present search report has been drawn up for all claims

| Place of search | Date of completion of the search | Examiner |
|---|---|---|
| The Hague | 22 May 2025 | Van Dop, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 3410

This annex lists the patent family members relating to the patent documents cited in the above-mentioned European search report.
The members are as contained in the European Patent Office EDP file on
The European Patent Office is in no way liable for these particulars which are merely given for the purpose of information.

22-05-2025

| Patent document cited in search report | Publication date | Patent family member(s) | Publication date |
|---|---|---|---|
| US 2018042503 A1 | 15-02-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019063861 A1

**Non-patent literature cited in the description**

- **RÍOS-MUÑOZ, G. R. et al.** Structural Remodeling and Rotational Activity in Persistent/Long-Lasting Atrial Fibrillation: Gender-Effect Differences and Impact on Post-Ablation Outcome. *Frontiers in Cardiovascular Medicine*, March 2022, vol. 9
- **RAPPEL W. J. et al.** Spatially Conserved Spiral Wave Activity during Human Atrial Fibrillation. *Circulation: Arrhythmia and Electrophysiology*, March 2024, vol. 17, E012041
- **HONARBAKHSH S. et al.** A Novel Mapping System for Panoramic Mapping of the LeftAtrium: Application to Detect and Characterize Localized Sources Maintaining AF. *JACC: Clinical Electrophysiology*, November 2017
- **HONARBAKHSH S. et al.** Characterization of drivers maintaining atrial fibrillation: Correlation with markers of rapidity and organization on spectral analysis. *Heart Rhythm*, September 2018, vol. 15, 1296-1303
- **NARAYAN S. M. et al.** Computational Mapping Identifies Localized Mechanisms for Ablation of Atrial Fibrillation. *PLoS ONE*, 2012, vol. 7 (9), e46034
- **QUINTANILLA J. G.** Instantaneous Amplitude and Frequency Modulations Detect the Footprint of Rotational Activity and Reveal Stable Driver Regions as Targets for Persistent Atrial Fibrillation Ablation. *Circulation Research*, August 2019, vol. 125, 609-627
- **SEITZ J. et al.** Artificial intelligence-adjudicated spatiotemporal dispersion: A patient-unique fingerprint of persistent atrial fibrillation. *Heart Rhythm*, January 2024

- **FRANCO E. et al.** Subjective identification and ablation of drivers improves rhythm control in patients with persistent atrial fibrillation. The CHAOS-AF study. *Revista española de cardiologia*, 2024, 2
- **BENALI K. et al.** Recurrences of Atrial Fibrillation Despite Durable Pulmonary Vein Isolation: The PARTY-PVI Study. *Circulation: Arrhythmia and Electrophysiology*, March 2023, vol. 16, E011354
- **RONEY C. H.** Constructing bilayer and volumetric atrial models at scale. *Interface Focus*, December 2023, vol. 13
- **JAFFERY O. A. et al.** A Review of Personalised Cardiac Computational Modelling Using Electroanatomical Mapping Data. *Arrhythmia and Electrophysiology Review*, 2024, vol. 13
- **RÍOS-MUÑOZ G. R. et al.** Real-Time Rotational Activity Detection in Atrial Fibrillation. *Frontiers in Physiology*, March 2018, vol. 9, 208
- **RÍOS-MUÑOZ G. R. et al.** Real-Time Ventricular Cancellation in Unipolar Atrial Fibrillation Electrograms. *Frontiers in Bioengineering and Biotechnology*, September 2020, vol. 8
- **SCHILLING C. et al.** *Non-Linear Energy Operator for the Analysis of Intracardial Electrograms*, 2009
- **RÍOS-MUÑOZ G. R. et al.** Hidden Markov Models for Activity Detection in Atrial Fibrillation Electrograms. *Computing in Cardiology Conference (CinC)*, December 2020, 1-4